Europäisches Patentamt

European Patent Office

Office européen des brevets

(19)

(11) Veröffentlichungsnummer : **0 139 945**
**B1**

(12) **EUROPÄISCHE PATENTSCHRIFT**

(45) Veröffentlichungstag der Patentschrift :
09.11.88

(21) Anmeldenummer : 84109629.0

(22) Anmeldetag : 13.08.84

(51) Int. Cl.⁴ : **B 01 J 19/26, B 01 J 14/00,**
**C 07 C 87/50, C 07 C139/00,**
**C 07 C143/24, C 07 C143/46,**
**C 07 C143/56**

(54) **Verfahren zur Herstellung von als Feststoffe anfallenden chemischen Verbindungen aus flüssigen Ausgangsstoffen.**

(30) Priorität : 24.08.83 DE 3330445

(43) Veröffentlichungstag der Anmeldung :
08.05.85 Patentblatt 85/19

(45) Bekanntmachung des Hinweises auf die Patenterteilung : 09.11.88 Patentblatt 88/45

(84) Benannte Vertragsstaaten :
BE CH DE FR GB IT LI

(56) Entgegenhaltungen :
DE-A- 2 752 197
DE-A- 2 813 570
DE-A- 3 022 256
GB-A- 1 500 343
US-A- 4 113 438
US-A- 4 389 375

(73) Patentinhaber : BAYER AG
Konzernverwaltung RP Patentabteilung
D-5090 Leverkusen 1 Bayerwerk (DE)

(72) Erfinder : Busse, Roland, Dipl.-Ing.
Fasanenweg 19
D-5090 Leverkusen 3 (DE)
Erfinder : Emde, Herbert, Dr.
Hardthofstrasse 13
D-5000 Köln 80 (DE)
Erfinder : Dürholz, Friedrich, Dr.
Kunsthöhe 40
D-5630 Remscheid 11 (DE)
Erfinder : Mayer, Dietmar, Dr.
In den Wiesen 1
D-5060 Bergisch Gladbach 2 (DE)
Erfinder : Jovcic, Dorde
Am Büscherhof 14
D-5653 Leichlingen (DE)

## Beschreibung

Die vorliegende Erfindung betrifft ein Verfahren zur Herstellung chemischer Verbindungen aus flüssigen Ausgangsstoffen, bei dem die chemische Verbindung als Feststoff anfällt.

Das Vermischen flüssiger Ausgangsstoffe, unter anderem zum Zweck der Umsetzung dieser Stoffe zu chemischen Verbindungen, ist eine wohlbekannte Grundoperation in der chemischen Technik. Dieses Vermischen flüssiger Ausgangsstoffe kann solange problemlos durchgeführt werden, als sich keine wesentlichen Änderungen des Aggregatzustandes ergeben. Ändert sich hingegen während des Vermischens und Umsetzens flüssiger Ausgangsstoffe der Aggregatzustand sehr stark, insbesondere infolge des Entstehens von Feststoffen, so treten schwerwiegende Probleme durch Verkrustungen, Anbackungen und Verstopfungen von Reaktoren und/oder Armaturen auf, die eine häufige Unterbrechung des Verfahrens erfordern oder in vielen Fällen Verfahren auf diesem Wege unmöglich machen.

Beispiele für solche Reaktionen sind die Bildung von Salzen aus Schmelzen organischer Säuren, wie organischer Sulfonsäuren, und flüssigen oder verflüssigten Alkalien, wie flüssigen, gegebenenfalls wasserhaltigen Alkalihydroxiden oder flüssigen oder verflüssigten Aminen. Hierbei bilden sich in einer fast augenblicklichen Reaktion die in den genannten flüssigen Ausgangsstoffen nicht oder ungenügend löslichen Salze der genannten organischen Säuren. Diese führen sodann zu den geschilderten Anbackungen oder Verstopfungen, so daß eine weitere Förderung der flüssigen Ausgangsstoffe verhindert oder zumindest stark gestört wird. Aber auch andere Umsetzungen von flüssigen Ausgangsstoffen, bei denen die entstehenden chemischen Verbindungen als Feststoffe anfallen, leiden unter den gleichen Schwierigkeiten.

So ist es notwendig, bei der Herstellung der Alkalisalze von aromatischen Hydroxyverbindungen zunächst die geschmolzene zugrundeliegende aromatische Sulfosäure mit flüssiger wäßriger Alkalilauge zur Reaktion zu bringen. Nach DE-OS 1 493 663 geschieht dies in einer rohrförmigen Mischkammer, in die die beiden flüssigen Ausgangsstoffe tangential eingeführt werden. Zur Vermeidung von Anbackungen befindet sich in dieser Mischkammer eine Vielkantwelle, so daß ein Spalt ausgebildet wird, in welchem die Durchmischung stattfindet und in welchem weiterhin Anbackungen durch die ununterbrochene Drehbewegung dieser Welle ständig entfernt werden. Eine solche Vorrichtung ist nicht nur aufwendig und teuer, da sie aus massivem Material bestehen muß und hohe Antriebskräfte verlangt, vielmehr müssen bei Störungen und beim Abfahren solcher mechanischen Mischeinrichtungen Spülvorgänge eingeleitet werden, da vielfach ein Aufschmelzen von restlichen Anbackungen nicht mehr möglich ist.

Weitere mechanische Vorrichtungen zur Herstellung von Alkalisalzen aromatischer Hydroxyverbindungen sind nach GB-A-939.570 eine Hammermühle oder hintereinandergeschaltete Kammerreaktoren (DE-A-2 813 570).

Der in GB-A-1 122 078 beschriebene Apparat zur Herstellung von Alkalimetallsalzen von aromatischen Hydroxyverbindungen besteht aus einer Zerstäuberscheibe und mechanischen Elementen zum Freihalten der Wände des unterhalb der Sprüheinrichtung befindlichen Kessels.

Es wurde nun ein Verfahren zur Herstellung von als Feststoffe anfallenden chemischen Verbindungen aus flüssigen Ausgangsstoffen gefunden, das dadurch gekennzeichnet ist, daß man die flüssigen Ausgangsstoffe aus einer konzentrischen Zweistoffdüse mit Ringspalt und einer in der Mitte vorgeschobenen Düse derart versprüht, daß die flüssigen Ausgangsstoffe in den sich durchdringenden Sprühkegeln vermischt werden und dabei zu Reaktion kommen, wobei der innere Sprühkegel einen um 1° bis 40 °C größeren Winkel hat als der äußere Sprühkegel, und daß man die entstandenen Feststoffpartikel in einem unterhalb der Düsen befindlichen Kessel in einem Suspensionsmittel auffängt.

Das erfindungsgemäße Verfahren eignet sich für die Durchmischung und Umsetzung flüssiger Ausgangsstoffe zur Bildung von als Feststoff anfallenden chemischen Verbindungen. Im allgemeinen wird man es mit zwei flüssigen Ausgangsstoffen zu tun haben. In Systemen, in denen mehr als zwei flüssige Ausgangsstoffe unter Bildung einer als Feststoff anfallenden chemischen Verbindung reagieren, wird es in vielen Fällen möglich sein, zwei oder mehrere der flüssigen Ausgangsstoffe, die miteinander noch nicht unter Bildung einer als Feststoff anfallenden chemischen Verbindung reagieren, durch Vorvermischung zu einer flüssigen Phase zu vereinigen und erst danach in erfindungsgemäßer Weise mit einem weiteren flüssigen Ausgangsstoff, der zur Bildung der als Feststoff anfallenden chemischen Verbindung führt, zu mischen und umzusetzen.

Infolge der Vermischung und Reaktion der flüssigen Ausgangsstoffe in den sich durchdringenden Sprühkegeln der getrennten Düsen wird ein Verkrusten und Verstopfen dieser Düsen vermieden. Ferner entsteht die als Feststoff anfallende chemische Verbindung in Form feiner Feststoffpartikel, die in einem unterhalb der Düsen befindlichen Kessel in einem Suspensionsmittel aufgefangen werden und hierbei eine gut rührbare Suspension ergeben. Wählt man nun die Entfernung zwischen den Düsen und der Oberfläche des genannten Suspensionsmittels sowie die räumliche Ausdehnung der Sprühkegel in geeigneter Weise, kann ein Auftreffen der Feststoffpartikel auf die senkrechten Wände des unter den Düsen befindlichen Kessels so gut wie völlig vermieden werden, so daß auch an den Wänden Anbackungen und entsprechend notwendig werdende Spülvorgänge vollkommen vermieden werden können.

Für den eigentlichen Misch- und Umsetzungsvorgang sind weder bewegte noch statische Mischeinrichtungen notwendig.

Die zur Anwendung kommende konzentrische Zweistoffdüse ist dem Fachmann bekannt.

Die Sprühkegel der beiden Düsen der konzentrischen Zweistoffdüse durchdringen sich möglichst weitgehend, ohne daß der Sprühkegel einer Düse die andere Düse trifft.

Bei dieser konzentrischen Zweistoffdüse tritt einer der beiden flüssigen Ausgangsstoffe durch einen ringförmigen Spalt aus und bildet dabei einen Sprühkegel. Der zweite flüssige Ausgangsstoff wird durch eine aus der Mitte dieses Ringspaltes austretende und etwas vorgebaute Düse versprüht, so daß sich innerhalb des ersten Sprühkegels ein zweiter Sprühkegel ausbildet. Beide Sprühkegel haben keine Berührung mit der Austrittsfläche des jeweils anderen Sprühkegels. Der innere Sprühkegel hat einen größeren Winkel als der äußere Sprühkegel. Beide Sprühkegel bilden im Durchdringungsbereich (Mischbereich) einen resultierenden Sprühkegel aus, bevor die Sprühkegel auf die Oberfläche einer vorgelegten Suspensions- bzw. Verdünnungsflüssigkeit treffen.

Beginnend mit dem Ort des Durchdringens der beiden Sprühkegel beginnt die Vermischung und Umsetzung der beiden flüssigen Ausgangsstoffe zu der in feinen Feststoffpartikeln anfallenden chemischen Verbindung.

In bevorzugter Weise wird das Zuführungsrohr zur Düse und/oder die Düsenaustrittsöffnung in einer dem Fachmann bekannten Weise so gestaltet, daß der austretende Kegel des flüssigen Ausgangsstoffes zusätzlich zu der in axialer Richtung fortschreitenden Bewegung eine tangentiale Bewegung entlang der Kegelaußenfläche erfährt. Die einzelnen Tröpfchen des kegelförmig versprühten flüssigen Ausgangsstoffes beschreiben somit auf der Kegeloberfläche eine schneckenförmige Linie, die sich entsprechend der Erweiterung des Kegels selbst auch erweitert. In besonders bevorzugter Weise wird für die beiden Sprühkegel ein verschiedener, im allgemeinen engegengesetzter Drall erzeugt. Dadurch verlaufen die soeben beschriebenen tangentialen Bewegungen an den Kegeloberflächen bei den verschiedenen Sprühkegeln in entgegengesetzter Richtung, so daß im Durchdringungsbereich der Sprühkegel eine noch intensivere Durchmischung der flüssigen Ausgangsstoffe erzielt wird.

Durch die dem Fachmann bekannte Detailgeometrie der Düse kann ein Kegelwinkel (Raumwinkel, das ist der Winkel zwischen den Mittellinien der einzelnen Sprühkegel) von nahe 0° bis nahe 180° ausgebildet werden. Ein Kegelwinkel von nahe 0 °C entspricht hierbei einem ohne besondere Ablenkung aus dem Zuführungsrohr des flüssigen Ausgangsstoffes austretendem Strahl. Ein Winkel von nahe 180 °C ist etwa einem sehr flachen vollständig geöffneten Regenschirm vergleichbar. Für die Durchführung des erfindungsgemäßen Verfahrens sind beide Grenzwerte wenig sinnvoll. Es ist vielmehr sinnvoll, einen Kegelwinkel im Sinne der gegebenen Erklärung dieses Kegelwinkels von beispielsweise 10 bis 120 °C, bevorzugt 15 bis 90 °C, besonders bevorzugt 20 bis 60 °C, einzustellen. Im Fall der konzentrischen Zweistoffdüse ist es erforderlich, zur Erzielung einer Durchmischung den inneren Sprühkegel mit einem größeren Winkel auszustatten als den von weiter oben kommenden äußeren Sprühkegel. Beispielsweise ist der Winkel des inneren Sprühkegels 1° bis 40 °C, bevorzugt 15 bis 30 °C, größer als der Winkel des äußeren Sprühkegels. Die genannten Winkel betreffen die Sprühkegel an der Austrittsstelle der Düse. Beginnend in der Durchdringungszone beider Sprühkegel bildet sich sodann ein resultierender Sprühkegel in der weiter oben bereits beschriebenen Weise. Der Winkel dieses resultierenden Sprühkegels liegt zwischen den verschiedenen Winkeln der ursprünglich Sprühkegel.

Die Geometrie des resultierenden Kegels wird ferner bestimmt durch die Schwerkraft der gebildeten Feststoffpartikel und ihre durch die Schwerkraft beeinflußte Bahn.

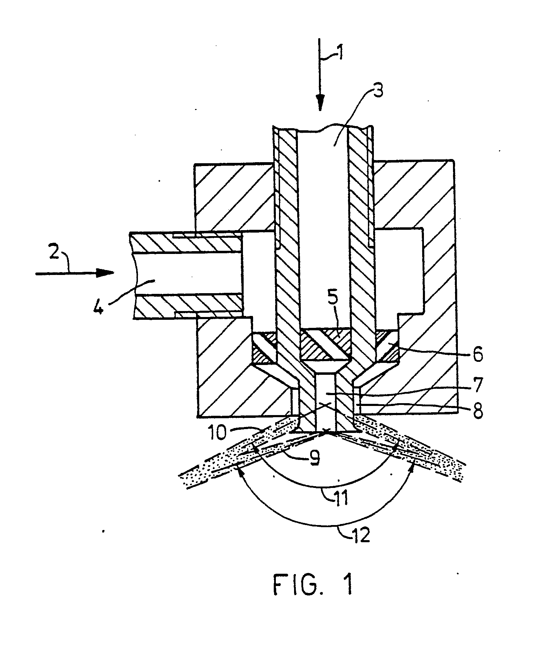

Der Aufbau einer Zweikomponenten-Düse sei beispielhaft in Figur 1 erläutert. Die Komponenten 1 und 2 werden jeweils in die Düsen 3 bzw. 4 eingeführt und durch die Drallkörper 5 bzw. 6 in den Düsenausgängen 7 bzw. 8 geleitet. Es bilden sich die Sprühkegel 9 und 10 aus. Die Kegel bilden die Raumwinkel 11 und 12.

In einer dem Fachmann bekannten Weise bildet sich der Sprühkegel dadurch aus, daß im Inneren des zugeführten Flüssigkeitsstrahles ein größerer Druck vorhanden ist als in dem Raum in den dieser Strahl durch die Düsenöffnung hineintritt. In Abhängigkeit von diesem Druckunterschied und weiterhin von der Viskosität des flüssigen Ausgangsstoffes sowie von der pro Zeiteinheit geförderten Flüssigkeitsmenge im Zusammenhang mit der Detailgeometrie der Düse bildet sich sodann der Sprühkegel, bestehend aus feinsten Flüssigkeitströpfchen, aus. Ein größerer Druckunterschied wird hierbei kleinere Tropfen erzeugen, jedoch auch einen höheren Energieaufwand zur Erzeugung dieses größeren Druckunterschiedes benötigen. Ein größerer Druckunterschied wird auch bei vorgegebener Düsengeometrie einen größeren Winkel des Sprühkegels hervorrufen und selbstverständlich einen größeren Durchsatz pro Zeiteinheit zulassen. Im Gegensatz hierzu wird ein sehr kleiner Druckunterschied beispielsweise den Sprühkegel zu einem zusammenhängenden Flüssigkeitsstrahl auch außerhalb der Düse degenerieren lassen. Übliche Druckunterschiede für die Durchführung des erfindungsgemäßen Verfahrens sind beispielsweise 0,5 bis 50 bar, bevorzugt 0,8 bis 30 bar, besonders bevorzugt 1 bis 16 bar. Entsprechend der weiter oben beschriebenen Möglichkeit, für die verschiedenen flüssigen Ausgangsstoffe verschiedene Sprühkegelwinkel einzustellen, ist es auch möglich, diese verschiedenen Winkel durch Anlegung verschiedener Druckdifferenzen an beiden Düsen oder Düsenaustrittsöffnungen einzustellen.

Der absolute Druck ist für die Durchführung des erfindungsgemäßen Verfahrens nicht wesentlich. Er kann von einem technisch und wirtschaftlich realisierbaren Unterdruck bis zu sehr hohen Drucken variieren, beispielsweise von 1 mbar bis 100 bar. Vielfach wird man das erfindungsgemäße Verfahren bei 0,1 bis 80 bar durchführen, in einer großen Anzahl von Anwendungsfällen kann jedoch bei Normaldruck gearbeitet werden.

Die Temperatur zur Durchführung des erfindungsgemäßen Verfahrens muß zwischen dem Schmelzpunkt und dem Siedepunkt bzw. Zersetzungspunkt der flüssigen Ausgangsstoffe liegen. Ihre absolute Höhe ist hierbei von den physikalischen Konstanten der flüssigen Ausgangsstoffe abhängig und kann daher nicht zahlenmäßig näher eingegrenzt werden. Diese Temperatur betrifft weiterhin nur die Zuführung des flüssigen Ausgangsstoffes, die Austrittsöffnungen der Düsen und die oberen Bereiche der Sprühkegel zur Erzeugung von Flüssigkeitströpfchen. Die weitere Einstellung im Durchdringungsbereich der verschiedenen Sprühkegel und darunter ist für die Durchführung des erfindungsgemäßen Verfahrens nicht kritisch und stellt sich im allgemeinen von selbst ein. Für diese sich von selbst einstellende Temperatur kommen folgende Randbedingungen in Frage :

a) Die Temperatur der herangeführten flüssigen Ausgangsstoffe,

b) die Reaktionswärme aufgrund der in der Durchdringungszone der Sprühkegel stattfindenden chemischen Umsetzung, beispielsweise der Neutralisation, und

c) der evtl. Wärme- und/oder Stoffabfuhr aus dem sich anschließenden Apparat.

Als Beispiele für die unter c) genannte Abfuhr von Wärme oder Stoffen sei darauf verwiesen, daß bei Einstellung von Normaldruck und einer höheren Temperatur als 100 °C die Abführung von beispielsweise Neutralisationswasser als Wasserdampf aus dem sich an die Vermischung anschließenden Reaktionsraum möglich ist. Hierdurch wird eine entstehende Suspension der als Feststoff anfallenden chemischen Verbindung in Bezug auf die noch verbleibende Wassermenge konzentriert. Als weiteres Beispiel sei erwähnt, daß bei Aufrechterhaltung eines höheren Druckes und Zurückhalten von Stoffen und der Wärmeenergie innerhalb des sich an die Vermischungszone anschließenden Reaktors die Temperatursteigerung durch die Neutralisationswärme für die Durchführung einer folgenden chemischen Reaktion, beispielsweise einer Umlagerung im Molekül der als Feststoff anfallenden chemischen Verbindung, genutzt werden kann.

Die Durchdringungszone der Sprühkegel dient somit als Misch- und Reaktionszone für die zugeführten flüssigen Ausgangsstoffe. Die hierbei in kleinen Feststoffpartikeln anfallende chemische Verbindung fällt sodann entsprechend der Schwerkraft dieser Feststoffpartikel nach unten und wird im allgemeinen in einem Suspensionsmittel aufgefangen, ohne daß vorher Wandberührung im wesentlichen Umfange mit den Wänden des die Suspensionsflüssigkeit enthaltenden Apparates eintritt. Als Suspensionsmittel kann hierbei jede geeignete Flüssigkeit vorgelegt werden, die in der gewünschten Weise eine rührfähige und transportfähige Suspension der als Feststoff anfallenden chemischen Verbindung ermöglicht. Im Falle der Neutralisation organischer Säuren durch Alkalisalzlösungen oder Amine kann dies beispielsweise Wasser sein, in dem sich die als Feststoff anfallenden Salze nicht oder nicht vollständig lösen, es kann weiterhin der Überschuß einer der flüssigen Komponenten sein, beispielsweise überschüssige geschmolzene organische Säure oder überschüssiges flüssiges Alkalihydroxid (gegebenenfalls als hochkonzentrierte Lösung) oder flüssiges Amin. Als Suspensionsmittel können jedoch auch ohne Beeinträchtigung der erfinderischen Idee inerte Lösungsmittel, wie aromatische oder aliphatische Kohlenwasserstoffe oder Halogenkohlenwasserstoffe, oder andere Suspensionsmittel eingesetzt werden, die gegen die als Feststoff anfallende chemische Verbindung oder gegen gegebenenfalls als Überschuß verbleibende flüssige Ausgangsstoffe inert sind.

Die aufgefangene Suspension kann beispielsweise durch ein Rührwerk stabil gehalten und vor dem Absitzen bewahrt werden. Entsprechend kann der unter den Düsen anzubringende Apparat ein Rührwerksbehälter sein. Hierbei kann die Suspension bis zu einer vorbestimmten max. Füllhöhe des Rührwerksbehälters zwischengelagert werden und dann chargenweise weiterverwendet werden, sie kann jedoch auch kontinuierlich durch einen Ablauf oder Überlauf für die Weiterverwendung entnommen werden. Des weiteren lassen sich liegende Behälter für das Auffangen und Zwischenlagern einer Suspension denken. Weiterhin ist es möglich, die Suspension in einem Trichter aufzufangen und durch eine Schlammpumpe oder durch einen Schneckenaustrag oder ähnliche Fördereinrichtungen abzuführen. Der unterhalb der Durchdringungszone der Sprühkegel anzubringende Behälter kann ferner seitlich oder oben einen Austritt für dampfförmig abzuziehende Stoffe, beispielsweise den Dampf des Neutralisationswassers, haben.

Im erfindungsgemäßen Verfahren werden flüssige Ausgangsstoffe durch Düsen zu Sprühkegeln versprüht. Als flüssige Ausgangsstoffe kommen hierbei reine Flüssigkeiten, Schmelzen von reinen Stoffen oder von Stoffgemischen, Lösungen, Emulsionen sowie Suspensionen in Betracht, wenn deren Teilchengröße es erlaubt, eine solche Suspension durch die Düse zu versprühen. Es ist sowohl möglich, jeden flüssigen Ausgangsstoff durch eine separate Düse zu versprühen. Da jedoch, wie bereits beschrieben, auch Mischschmelzen, Lösungen, Emulsionen oder Suspensionen, d. h. Mehrstoffsysteme durch eine Düse versprüht werden können, ist es im Prinzip auch möglich, bei mehr als zwei flüssigen Ausgangsstoffen zwei oder mehrere dieser flüssigen Ausgangsstoffe vor der Versprühung zu vermischen, falls diese noch nicht zu einer als Feststoff anfallenden chemischen Verbindung reagieren, und dann als Mischung mehrerer flüssiger Ausgangsstoffe gemeinsam zu versprühen und den kritischen weiteren flüssigen Ausgangsstoff, der dann zu der als Feststoff anfallenden chemischen Verbindung reagiert, in einer weiteren getrennten Düse zu versprühen.

In hervorragender Weise eignet sich das erfindungsgemäße Verfahren zur Herstellung von Salzen organischer Säuren, beispielsweise Carbonsäuren, Sulfonsäuren oder Phosphonsäuren, mit Metallionen, beispielsweise Alkaliionen in höher konzentrierter Form. Will man solche Salze nach herkömmlichen Verfahren aus Schmelzen oder hochkonzentrierten Lösungen der Säure und Metallionen in hochkonzentrierter flüssiger Form herstellen, so verfestigen sich die gebildeten Salze sofort zu einer harten Masse, die die Reaktoren, wie Mischrohre oder Rührgefäße in kürzester Zeit zusetzen und zur Unterbrechung der Reaktion zwingen. In einer weiteren hervorragenden Weise eignet sich das erfindungsgemäße Verfahren zur Herstellung der Salze von gegebenenfalls substituiertem Anilin und einer Mineralsäure, bevorzugt Schwefelsäure. Eine noch weitere hervorragende Eignung ist bei der Herstellung von in Wasser schwerlöslichen organischen Säuren gegeben, wenn die in Wasser leicht löslichen Alkalisalze solcher organischen Säuren mit Mineralsäure umgesetzt werden. Die genannten schwierigen verfahrenstechnischen Situationen entstehen jedoch grundsätzlich auch bei allen anderen Reaktionen, bei denen die entstehenden chemischen Verbindungen als Feststoffe anfallen, bei denen also infolge der hohen gewünschten Konzentration des entstehenden Stoffes zu wenig Lösungsmittel vorhanden ist, um eine homogene flüssige Phase auszubilden. Zur Illustration seien beispielsweise die folgenden Einzelfälle näher ausgeführt :

Zur Herstellung von 3-Hydroxybenzoesäure wird Benzoesäure zunächst mit Schwefelsäure und/oder gasförmigem oder flüssigem SO$_3$ zur 3-Sulfobenzoesäure sulfoniert. Diese 3-Sulfobenzoesäure wird sodann in ihr Alkalisalz, beispielsweise in ihr Natriumsalz, übergeführt und mit überschüssigem Alkalihydroxid, beispielsweise Natriumhydroxid, unter Austritt der Sulfonsäuregruppe zum Natriumsalz der 3-Hydroxybenzoesäure verschmolzen. Die Herstellung dieses gewünschten Alkalisalzes der 3-Sulfobenzoesäure, gegebenenfalls im Gemisch mit dem in der folgenden Alkalischmelze erforderlichen überschüssigen Alkalihydroxid kann nun in besonders günstiger Weise mit dem erfindungsgemäßen Verfahren durchgeführt werden, ohne daß es zu Verklumpungen oder Verstopfungen durch das gebildete Salz kommt. Hierzu wird beispielsweise in einem Rührkessel mit den üblichen Einrichtungen für Temperatur- und Druckmessung gearbeitet. Im Raum zwischen dem Deckel des Rührkessels und dem Rührer und oberhalb des später zu erwartenden Flüssigkeitsstandes der entstehenden Suspension wird die konzentrische Zweistoffdüse für die zuzuführenden flüssigen Ausgangsstoffe angebracht.

Für den Fall, daß in der üblichen Weise das Rührwerk von oben in den Kessel eingeführt ist, wird diese konzentrische Zweistoffdüse exzentrisch im Rührkessel zwischen Rührerwelle und Wandung angebracht. Die Sprühwinkel der austretenden flüssigen Ausgangsstoffe werden so eingestellt, daß im wesentlichen keine Wandberührung und keine Berührung der Rührerwelle erfolgt. Ein geringes Verspritzen ist nicht kritisch, da das als kleine Feststoffpartikel anfallende Salz der 3-Sulfobenzoesäure in dieser Form leicht von der Wandung und von der Rührerwelle abgespült werden kann. Dieses Abspülen erfolgt im einfachsten Fall durch das Herumspritzen der durch den Rührer bewegten Suspension im Kessel bzw. im unteren Bereich des Kessels infolge des ansteigenden Flüssigkeitsspiegels der entstehenden Suspension. Als Suspensionsmittel kann eine kleine Menge Wasser, die zur Bildung einer Lösung nicht ausreicht, oder eine kleine Menge der Lösung des verwendeten Hydroxids eingesetzt werden. Im einfachsten Fall und daher in bevorzugter Weise wird kein Suspensionsmittel gesondert vorgelegt, sondern das im Verlaufe der Neutralisationsreaktion sich bildende Neutralisationswasser hierzu verwendet. Als der eine der flüssigen Ausgangsstoffe wird geschmolzene 3-Sulfobenzoesäure bzw. in bevorzugter Form eine technische Sulfierschmelze der 3-Sulfobenzoesäure eingesetzt. Eine solche technische Sulfierschmelze kann beispielsweise die folgende Zusammensetzung haben :

70 bis 95 Gew.-% 3-Sulfobenzoesäure,
2,5 bis 7 Gew.-% 4-Sulfobenzoesäure,
0,5 bis 1,5 Gew.-% 2-Sulfobenzoesäure,
0,01 bis 0,5 Gew.-% 3,5-Disulfobenzoesäure,
0,01 bis 1,5 Gew.-% Diphenylsulfon-Derivate,
0,01 bis 1,5 Gew.-% Benzophenon-Derivate und
ca. 2,0 bis 20 Gew.-% SO$_3$ (in Form von SO$_3$ und/oder H$_2$SO$_4$).

Der andere flüssige Ausgangsstoff ist 50- bis 100-gew.-%iges Alkalihydroxid, wobei der Rest von 50 bis 0 Gew.-% im wesentlichen aus Wasser besteht. In bevorzugter Weise wird 60- bis 90-%iges, insbesondere 65- bis 80-%iges Alkalihydroxid eingesetzt. Als Alkalihydroxid kommt beispielsweise Natriumhydroxid und Kaliumhydroxid, bevorzugt Natriumhydroxid, in Frage.

Als Temperatur für die flüssigen Ausgangsstoffe kommt der Bereich von 20 bis 350 °C, bevorzugt 90 bis 200 °C, in Frage.

Da für die sich an die Salzbildung anschließende Alkalischmelze eine größere als die stöchiometrisch zur Salzbildung erforderliche Alkalihydroxidmenge mit der 3-Sulfobenzoesäure bzw. dem Sulfiergemisch vermischt wird, wird die Menge an Alkalihydroxid so bemessen, daß nach der Neutralisation aller Sulfo- und Carboxygruppen und im Falle der Sulfierschmelze auch nach Neutralisation der Schwefelsäure pro Mol 3-Sulfobenzoesäure noch weitere 2,5 bis 8 Mol, bevorzugt 3 bis 6 Mol, besonders bevorzugt 3,5 bis 5,5 Mol, ganz besonders bevorzugt 4 bis 5 Mol Alkalihydroxid, vorliegen.

Die genannten flüssigen Ausgangsstoffe werden in getrennten Rührbehältern flüssig vorgehalten und über Dosierpumpen der konzentrischen Zweistoffdüse zugeführt und in den Gasraum des Rührkessels versprüht. In bevorzugter Weise wird hierbei die Säure bzw. die saure Sulfierschmelze über die zentral gelegene hervorstehende Düse mit einem größeren Sprühkegelwinkel versprüht, während das flüssige Alkalihydroxid über den außen angebrachten Ringspalt mit einem kleineren Sprühkegelwinkel als der Winkel des inneren Sprühkegels versprüht wird. Hierdurch wird in besonderer Weise der Korrosion aller Apparateteile durch die Säure bzw. die saure Sulfierschmelze vorgebeugt, da alle sauren Bestandteile aus dem inneren Sprühkegel vor einer Wandberührung oder einer Berührung der Rührerwelle immer erst durch den äußeren alkalischen Sprühkegel hindurchtreten müssen und dabei neutralisiert und unschädlich gemacht werden.

Weiterhin kann die Salzbildung in dieser Reaktion durch Anlegen eines erhöhten Druckes im Rührkessel von beispielsweise 30 bis 80, bevorzugt 40 bis 60 bar, so gelenkt werden, daß das Neutralisationswasser nur in untergeordnetem Maße verdampft und daher die Neutralisationswärme voll in der Suspension des Salzes erhalten bleibt. Dadurch entfällt das Aufheizen der Suspension für die folgende Alkalischmelze weitgehend, so daß das ganze Verfahren unter Berücksichtigung der folgenden Stufe äußerst günstig betrieben werden kann.

Als Suspensionsmittel dient in diesem Falle das überschüssige Alkalihydroxid.

In einem weiteren Beispiel sei auf die Herstellung von 4,4'-Dihydroxy-diphenyl hingewiesen, wobei aus Diphenyl durch Sulfonierung zunächst die Diphenyl-4,4'-disulfonsäure gebildet wird, die durch Salzbildung in das Dialkalisalz übergeführt wird und anschließend in Gegenwart von überschüssigem Alkalihydroxid unter Eliminierung der Sulfonsäuregruppen in das Dialkalisalz des 4,4'-Dihydroxy-diphenyls übergeführt wird.

In ähnlicher Weise, wie soeben für die 3-Hydroxy-benzoesäure ausgeführt, wird auch hier von der geschmolzenen Disulfonsäure oder in bevorzugter Weise von einem technischen Sulfiergemisch ausgegangen. Ein solches technisches Sulfiergemisch kann beispielsweise folgende Zusammensetzung haben :

mindestens 30 Gew.-%, beispielsweise 30 bis 80 Gew.-%, Diphenyl-4,4'-disulfonsäure,
0 bis 8 Gew.-% Diphenyl-3,4'-disulfonsäure,
0 bis 5 Gew.-% Diphenyl-3,3'-disulfonsäure,
0 bis 2 Gew.-% Diphenyl-4-sulfonsäure,
0 bis 54 Gew.-% Schwefelsäure und
30 bis 1 Gew.-% Wasser.

Das Alkalihydroxid, bevorzugt Natriumhydroxid, wird auch hierbei in einer Konzentration von mindestens 50 Gew.-% Alkalihydroxid eingesetzt, wobei der Rest im wesentlichen Wasser ist. Beispielsweise sei eine Konzentration von 50 bis 96 Gew.-%, bevorzugt von 60 bis 95 Gew.-%, besonders bevorzugt 65 bis 85 Gew.-%k, Alkalihydroxid genannt. Die Menge des Alkalihydroxids, die über die zur Neutralisation aller Säuregruppen in der eingesetzten Disulfonsäure bzw. im eingesetzten Sulfiergemisch hinaus anwesend sein soll, beträgt 8 bis 24 Mol Alkalihydroxid pro Mol Diphenyldisulfonsäure, bevorzugt 11 bis 17 Mol Alkalihydroxid.

Als Temperatur für die eingesetzten Ausgangsstoffe kommen die bereits genannten in Frage. In bevorzugter Weise wird auch hier wieder der saure flüssige Ausgangsstoff über die zentrale Düse versprüht. Auch hier kann zur Erhaltung der Neutralisationswärme in der entstehenden Suspension der Druck im Rührkessel auf einen höheren Wert angesetzt werden. Zur weiteren Konzentration der Suspension des Dialkalisalzes kann jedoch auch bei Normaldruck Wasserdampf aus dem Rührkessel abgezogen werden.

In grundsätzlich gleicher Weise kann verfahren werden, wenn zur Bildung von Monohydroxy-diphenyl von der Diphenyl-monosulfonsäure ausgegangen wird und über die Alkalisalzbildung und Alkalischmelze weiter verfahren wird.

Die 3-Hydroxybenzoesäure ist ein wichtiges Zwischenprodukt zur Herstellung von Pflanzenschutzmitteln. So kann nach US 4 031 131 3-Hydroxy-benzoesäure in methanolischer Lösung und in Gegenwart von Kaliumhydroxid und Dimethylsulfoxid mit 3,4-Dichlorbenzotrifluorid zur 3-(2-Chlor-4-trifluormethylphenoxy)-benzoesäure umgesetzt werden, die durch Nitrieren mit Kaliumnitrat in konzentrierter Schwefelsäure weiter in die 5-(2-Chlor4-trifluormethyl-phenoxy)-2-nitrobenzoesäure umgesetzt wird, die nach US 3 798 276 ein wichtiges Herbizid darstellt.

Dihydroxy-diphenyle sind ein Ausgangsmaterial für hochwertige Kondensationspolymere, wie Polycarbonate und Polyester, wobei besonders die Eigenschaft der Hochtemperatur-Beständigkeit bedeutsam ist (DE-OS 3 031 094). Dihydroxy-diphenyle finden ferner Verwendung als Zwischenprodukt für pharmazeutische Produkte sowie als Stabilisator und Antioxidantien für Kautschuke, Öle und Polymere.

Das 4-Hydroxy-diphenyl (p-Phenyl-phenol) wird als Kettenabbrecher zur Einstellung der Molekulargewichte für die genannten Polymeren aus Dihydroxy-diphenyl eingesetzt und ist ferner ein Zwischenprodukt zur Herstellung von Lackharzen, nichtionogenen Emulgatoren und Pflanzenschutzmitteln (Ullmanns Enzyklopädie der Technischen Chemie, 4. Auflage, Band 18, S. 219).

Beispielhaft für weitere Anwendungen sollen die folgenden Sulfonsäuren genannt werden, die in die entsprechenden Hydroxyverbindungen überführt werden : Naphthalin-1- und -2-sulfosäure (ergeben α- und β-Naphthol), Naphthalin1.5-di-sulfosäure (ergibt Azurinsäure = 1-Hydroxynaphthalin-5-sulfonsäure oder Azurol = 1,5-Dihydroxynaphthalin), 1,3,6-Trisulfosäure des Naphthalins (1-OH-9.6-Disulfosäure), Benzoldisulfosäure (Resorcin), Benzolsulfonsäure (Phenol), Toluolsulfonsäure (Kresol), Carbazoltetrasulfosäure (Oxycarbazoltrisulfosäure).

Weitere Einsatzmaterialien sind beispielsweise die isomeren Naphthalin-di- und -trisulfosäuren, Aminonaphthalinmono-, -di- und -trisulfosäuren und Hydroxinaphthalinmono-, -di- und -trisulfosäuren.

Als ein weiteres Beispiel für die erfolgreiche Durchführung des erfindungsgemäßen Verfahrens sei die Herstellung von Aminoaryl-sulfonsäuren genannt, bei der aus einem gegebenenfalls substituierten aromatischen Amin und Schwefelsäure zunächst das gegebenenfalls substituierte Amin-hydrogensulfat gebildet wird, welches im sogenannten Back-Verfahren in die Aminoarylsulfonsäure umgesetzt wird. Als einen der flüssigen Ausgangsstoffe setzt man hierbei ein gegebenenfalls im aromatischen Kern und/oder an dem Amino-Stickstoff substituiertes aromatisches Amin ein, dessen aromatischer Kern ein gegebenenfalls substituiertes Benzol-, Naphthalin-, Anthracen-, Naphthochinon- oder Anthrachinon-Gerüst oder das Gerüst eines aromatischen Heterocyclus sein kann und das am Amino-Stickstoff ein- oder zweifach durch Alkyl, Aralkyl oder Aryl substituiert sein kann, wobei weiterhin das Stickstoffatom mit den Substituenten einen Stickstoffheterocyclus bilden kann. In bevorzugter Weise ist das aromatische Gerüst das Benzol- oder Naphthalingerüst, in besonders bevorzugter Weise das Benzolgerüst. Der Prototyp dieser Reaktion ist die Umsetzung des nicht substituierten Anilins zu Anilin-hydrogensulfat und weiter zu Sulfanilsäure.

Als weiteren flüssigen Ausgangsstoff setzt man Schwefelsäure ein, die einen Wassergehalt von bis zu 30 Gew.-% haben kann. In bevorzugter Weise wird 96 bis 100 gew.-%ige Schwefelsäure und in ganz besonders bevorzugter Weise 100 %ige Schwefelsäure (Monohydrat) eingesetzt.

Da die Schmelzpunkte der in diesem Beispiel genannten flüssigen Ausgangsstoffe sehr niedrig liegen, kann auch die Temperatur der zugeführten flüssigen Ausgangsstoffe niedriger liegen als bei den vorgenannten Beispielen. Als Temperaturbereich sei beispielsweise 0 bis 100 °C, bevorzugt 15 bis 80 °C, genannt. Da für das anschließende Backverfahren ein Überschuß eines der beiden Ausgangsstoffe nicht erforderlich ist, wird man im allgemeinen das Molverhältnis des Arylamins und der Schwefelsäure nahe beim Äquivalenzpunkt einstellen, beispielsweise 0,95 bis 1,05 Mol Schwefelsäure pro Mol Arylamin, bevorzugt 0,98 bis 1,05, besonders bevorzugt nahe 1.

Wählt man die Temperatur der beiden Ausgangsstoffe höher, beispielsweise 120-180 °C, und dosiert diese beiden Stoffe in heißes vorgelegtes Suspensionsmittel, so kann die bei der Vermischung auftretende Neutralisationswärme ausgenutzt werden, um über das Arylaminhydrogensulfat in einem Schritt die Arylaminsulfonsäure herzustellen. Die entstandene Suspension kann beispielsweise durch Filtration oder durch wäßrige alkalische Extraktion aufgearbeitet werden.

Als Apparatur kann der o. g. Rührkessel mit exzentrisch eingesetzter konzentrischer Zweistoffdüse benutzt werden. Im Sinne einer Korrosionsverhinderung kann auch hier der innere Kegel aus der Schwefelsäure gebildet werden, während der äußere Kegel aus dem flüssigen, geschmolzenen oder gegebenenfalls in einem Lösungsmittel gelösten Arylamin gebildet wird.

Die Aminoarylsulfonsäuren sind wertvolle Zwischenprodukte für die Herstellung von Pharmazeutika, Schaumstoffen, optischen Aufhellern, Netzmitteln, synthetischen Beizmitteln, Gerbstoffen, Reservierungsmitteln, Insektiziden, Appreturmitteln, Weichmachern und polymeren Verdickungsmitteln (Ullmanns Enzyklopädie der Technischen Chemie, 3. Auflage, Band 16, S. 561).

Die für den erfolgreichen Ablauf des Backverfahrens und die Erzielung einer hohen Qualität der Aminoarylsulfonsäure erforderliche Homogenität des zunächst gebildeten Arylammoniumhydrogensulfats wird im erfindungsgemäßen Verfahren in besonders hoher Vollkommenheit erreicht.

Ein noch weiteres Beispiel der Anwendung des erfindungsgemäßen verfahrens ist die Gewinnung der freien H-Säure (1-Amino-8-hydroxynaphthalin-3,6-disulfonsäure) durch Umsetzung des Natriumsalzes mit Schwefelsäure oder einer anderen Mineralsäure. So fällt bei der Herstellung der H-Säure aus dem Trinatriumsalz der 1-Aminonaphthalin-3,6,8-trisulfonsäure und heißer Natronlauge eine wäßrige Lösung an, die hauptsächlich H-Säure-Na-salz, Natriumsulfit und überschüssiges NaOH enthält und die mit Schwefelsäure unter $SO_2$-Entwicklung ungesetzt wird (Ullmanns Enzyklopädie der technischen Chemie, 4. Aufl., Band 17, S. 104). Die wäßrige Lösung und die Schwefelsäure werden in der obenbeschriebenen Weise aus der konzentrischen Zweistoffdüse so versprüht, daß sich deren Sprühkegel durchdringen, wobei die freie H-Säure als Feststoff entsteht, die dann in der sauren Lösung der entstehenden Salze als Suspensionsmittel aufgenommen wird.

Die folgende Zusammenstellung enthält die wichtigsten Vorteile des erfindungsgemäßen Verfahrens:

1. Es sind keine mechanischen Mischer oder Mischeinrichtungen notwendig ; dadurch wird ein Anbacken des entstehenden Reaktionsgemisches vermieden, so daß auf Spüleinrichtungen verzichtet werden kann.

2. Die Reaktionswärme beispielsweise einer Neutralisationsreaktion kann entweder zur Temperatursteigerung der aufgefangenen Suspension oder zum Ausdampfen von überschüssigem Wasser oder Lösungsmittel genutzt werden.

3. Durch die Feinstverteilung des Gemisches im freien Raum wird eine große Oberfläche geschaffen, die gute Voraussetzungen für das schaumfreie Abdestillieren von Wasser, Lösungsmittel oder flüchtigen Nebenkomponenten bildet.

4. Die Feinstverteilung und innige Durchmischung zur Reaktion der flüssigen Ausgangsstoffe findet im freien Raum statt, wodurch Korrosionsprobleme durch aggressive Reaktionspartner, beispielsweise durch freie Säuren, nicht zu befürchten sind.

5. Die als Feststoff anfallende chemische Verbindung entsteht in Form von feinen Feststoffpartikelchen, so daß eine feinstverteilte, gut rührbare und gut förderbare Suspension entsteht, die alle nachfolgenden Bearbeitungsschritte erleichtert.

6. Durch die gleichmäßige, simultane und feinstverteilte Durchmischung der Reaktionspartner werden örtliche Überhitzungen verhindert.

7. Gegenüber den bekannten mechanischen Mischeinrichtungen hat das erfindungsgemäße Verfahren den Vorteil kleiner Einbaumaße der erforderlichen Düsen und kann daher in vorhandenen Apparaten leicht nachträglich eingebaut und durchgeführt werden.

8. Durch die Möglichkeit, die Neutralisionswärme innerhalb der entstehenden Suspension zu halten, kann die Aufheizzeit für eine etwa nachfolgende Schmelzreaktion verkürzt werden oder ganz entfallen.

9. Für den Fall einer Neutralisation und Bildung von Salzen organischer Säuren zum Zwecke einer nachfolgenden Schmelzreaktion werden die getrennten Verfahrensschritte der Neutralisation, der Trocknung sowie der Zwischenlagerung des getrockneten Salzes und sein Transport und Eintrag in den Schmelzreaktor eingespart bzw. zusammengefaßt.

10. Im Falle der Herstellung des Dialkalisalzes von Diphenyl-disulfonsäure zum Zweck der anschließenden Alkalischmelze zur Herstellung von Dihydroxy-diphenyl kann wegen der Entstehung der feinstverteilten, gut rührbaren Suspension im erfindungsgemäßen Verfahren der Natronlaugeneinsatz von 20 Mol pro Mol Disulfonsäure bei Verfahren nach dem Stande der Technik auf 8-24 Mol, bevorzugt auf 11-17 Mol gesenkt werden.

11. Die Herstellung von Aminoarylsulfonsäuren in einem Suspensionsmittel kann ohne mögliche Anbackung sowie ohne chemische Reaktion eines der Ausgangsstoffe mit dem Suspensionsmittel durchgeführt werden.

Beispiel 1

Als Reaktionsapparatur wurde ein 20 l-Rührwerksautoklav mit zentral von oben eingeführtem Rührwerk und exzentrisch angeordneter konzentrischer Zweistoffdüse benutzt. Innerhalb von 15 Minuten wurden simultan 8,5 kg eines technischen Sulfiergemisches mit 84 Gew.-% 3-Sulfobenzoesäure mit einer Temperatur von 100 °C und einem Druck von 40-60 bar und 14,2 kg 74 %ige wäßrige Natronlauge mit einer Temperatur von 100 °C und einem Druck von 40-60 bar über diese Zweistoffdüse versprüht, wobei die Säure über die innere Düse und die Lauge über den äußeren Ringspalt versprüht wurden. Im Rührkessel wurde ein Druck von 30-50 bar und eine Temperatur von 320-330 °C eingestellt. Das Neutralisationswasser und die überschüssige Natronlauge bildeten das Suspensionsmittel, dessen Oberfläche innerhalb des Rührkessels im Verlaufe des Versuches anstieg. Der innere Sprühkegel hatte etwa einen Winkel von 60°, der äußere Sprühkegel einen Winkel von 30°, so daß sich, beginnend von der Durchdringungszone, ein resultierender Winkel von etwa 45° einstellte. Die Sprühhöhe von der Düse zur Oberfläche der Suspension betrug anfangs 55 cm, zum Schluß des Versuches 12 cm.

Beispiel 2

In einem 100 l-Rührwerkskessel mit 600 mm Durchmesser, der in analoger Weise wie in Beispiel 1 ausgerüstet war, wurden simultan 44,3 kg/h Diphenyldisulfosäureschmelze mit einem Gehalt von 65,6 Gew.-% Diphenyl-4,4'-disulfonsäure mit einer Temperatur von 100 °C und einem Druck von 4 bar und 44,3 kg/h 74 gew.-%iger wäßriger Natronlauge mit einer Temperatur von 100 °C und einem Druck von 4 bar über eine konzentrische Zweistoffdüse in den bei einem Druck von 1 bar gehaltenen Rührkessel versprüht, wobei die saure Schmelze durch die innere Düse und die Natronlauge durch den äußeren Ringspalt geführt wurde. Das verdampfende Wasser wurde kondensiert und floß in den Kessel zurück. Im Kessel stellte sich eine Temperatur von 145-147 °C ein. Die Sprühhöhe betrug anfangs ca. 55 cm und zum Schluß ca. 25 cm. Läuft das Kondensat nicht in den Kessel zurück, stellt sich eine Kesseltemperatur von 165 °C-170 °C ein.

Beispiel 3

In einer Apparatur wie in Beispiel 2 wurden simultan 40 kg/h einer etwa 30 gew.-%igen wäßrigen Lösung von H-Säure-trinatriumsalz, NaOH und $Na_2SO_3$ und 13 kg/h etwa 60 %ige $H_2SO_4$ eingedüst (Sprühwinkel : 45° außen, 75° innen) ; die $H_2SO_4$ wurde durch die innere Düse geführt. Durch die Neutralisationswärme stieg die Temperatur der mit etwa 40-80 °C eingedüsten Stoffströme auf etwa 100 °C in der Suspension an, wodurch gleichzeitig das entstehende $SO_2$ völlig ausgaste. Die im Kesselsumpf entstandene Suspension von H-Säure in der sauren Salzlösung konnte ohne Schwierigkeiten im Maße ihres Entstehens aus dem Kessel abgezogen und zur weiteren Aufarbeitung gefördert werden.

**Patentansprüche**

1. Verfahren zur Herstellung von als Feststoff anfallenden chemischen Verbindungen aus flüssigen Ausgangsstoffen, dadurch gekennzeichnet, daß man die flüssigen Ausgangsstoffe aus einer konzentrischen Zweistoffdüse mit Ringspalt und einer in der Mitte vorgeschobenen Düse derart versprüht, daß die flüssigen Ausgangsstoffe in den sich durchdringenden Sprühkegeln vermischt werden und dabei zur Reaktion kommen, wobei der innere Sprühkegel einen um 1° bis 40° größeren Winkel hat als der äußere Sprühkegel, und daß man die entstandenen Feststoffpartikel in einem unterhalb der Düsen befindlichen Kessel in einem Suspensionsmittel auffängt.

2. Verfahren nach Anspruch 1, dadurch gekennzeichnet, daß der Winkel der Sprühkegel 5 bis 120 °C beträgt.

3. Verfahren nach Ansprüchen 1 und 2, dadurch gekennzeichnet, daß in der Düse ein Drall des Sprühkegels erzeugt wird.

4. Verfahren nach Ansprüchen 1 bis 3, dadurch gekennzeichnet, daß der Drall beider Sprühkegel in gegenläufiger Richtung erzeugt wird.

5. Verfahren zur Herstellung von als Feststoff anfallenden Alkalisalzen aromatischer Sulfosäuren durch Reaktion von Sulfosäuren mit Alkalihydroxid nach Anspruch 1, dadurch gekennzeichnet, daß man geschmolzene Sulfosäuren mit flüssigem Alkalihydroxid mittels einer konzentrischen Zweistoffdüse mit außerhalb der Düsen stattfindender Vermischung und Neutralisation umsetzt.

6. Verfahren nach Anspruch 5 zur Herstellung einer für die Alkalischmelze zum 4,4'-Dihydroxydiphenyl geeigneten Diphenyl-4,4'-disulfonsäure-Na-salz enthaltenden Suspensionen, dadurch gekennzeichnet, daß man als Sulfosäure das technische Sulfiergemisch mit mindestens 30 Gew.-% Diphenyl-4,4'-disulfonsäure und als Alkalihydroxid mindestens 50 gew.-%ges wäßriges Natriumhydroxid in einer Menge von etwa 8 bis 24 Mol NaOH pro Mol Diphenyl-4,4'-disulfonsäure-Nasalz nach Neutralisation aller sauren Gruppen einsetzt.

7. Verfahren zur Herstellung von Arylammonium-hydrogensulfaten aus Arylamin und Schwefelsäure nach Anspruch 1, dadurch gekennzeichnet, daß man geschmolzenes oder durch Zusatz von Lösungsmitteln verflüssigtes Arylamin und gegebenenfalls wasserhaltige Schwefelsäure mittels einer konzentrischen Zweistoffdüse mit außerhalb der Düsen stattfindender Vermischung und Neutralisation umsetzt.

8. Verfahren zur Herstellung von Aminoarylsulfonsäuren aus Arylaminen und Schwefelsäuren nach Ansprüchen 1 und 7, dadurch gekennzeichnet, daß man geschmolzenes oder durch Zusatz von Lösungsmitteln verflüssigtes Arylamin und gegebenenfalls wasserhaltige Schwefelsäure mittels einer konzentrischen Zweistoffdüse mit außerhalb der Düsen stattfindender Vermischung und Neutralisation umsetzt und daß man durch Aufrechterhalten einer Temperatur von 120-180 °C die Reaktion in einem Schritt über das Arylammonium-hydrogensulfat hinausgehen läßt, um die Aminoarylsulfonsäure zu erhalten.

9. Verfahren nach Ansprüchen 5 bis 8, dadurch gekennzeichnet, daß man in der konzentrischen Zweistoffdüse die saure Komponente durch die zentrale Düse und die alkalische Komponente durch den Ringspalt versprüht.

**Claims**

1. Process for the preparation of chemical compounds obtained as solids from liquid starting substances, characterized in that liquid starting substances are sprayed from a concentric two-component nozzle with an annular gap and a nozzle inserted in the middle such that the liquid starting substances are mixed in the spray cones which penetrate each other, and thereby react, the inner spray cone having an angle which is 1° to 40° greater than that of the outer spray cone, and in that the solid particles formed are collected in a suspending agent in a kettle located below the nozzle.

2. Process according to Claim 1, characterized in that the angle of the spray cone is 5 to 120°.

3. Process according to Claims 1 and 2, characterized in that a spiral thread of the spray cone is produced in the nozzle.

4. Process according to Claims 1 to 3, characterized in that the spiral thread of both spray cones is produced in opposite directions.

5. Process for the preparation of alkali metal salts, obtained as a solid, of aromatic sulphonic acids by reaction of sulphonic acids with an alkali metal hydroxide according to Claim 1, characterized in that molten sulphonic acids are reacted with liquid alkali metal hydroxide by means of a concentric two-component nozzle, with mixing and neutralization taking place outside the nozzles.

6. Process according to Claim 5 for the preparation of a suspension which contains a diphenyl-4,4'-disulphonic acid Na salt which is suitable for alkaline fusion to give 4,4'-dihydroxydiphenyl, characterized in that a technical grade sulphonation mixture containing at least 30 % by weight of diphenyl-4,4'-disulphonic acid is used as the sulphonic acid, and at least 50 % strength by weight of aqueous sodium hydroxide is used as the alkali metal hydroxide, in an amount of about 8 to 24 mole of NaOH per mole of diphenyl-4,4'-disulphonic acid Na salt, after neutralization of all the acid groups.

7. Process for the preparation of arylammonium hydrogen sulphates from arylamine and sulphuric acid according to Claim 1, characterized in that molten arylamine or arylamine which has been liquefied by the addition of solvents, sulphuric acid, if appropriate containing water, are reacted by means of a concentric two-component nozzle with mixing and neutralization taking place outside the nozzles.

8. Process for the preparation of aminoaryl sulphonic acids from arylamines and sulphuric acids, according to Claims 1 and 7, characterized in that molten arylamine or arylamine which has been liquefied by the addition of solvents, and sulphuric acid, if appropriate containing water, are reacted by means of a concentric two-component nozzle with mixing and neutralization taking place outside the nozzles, and in that, by maintaining a temperature of 120-180 °C, the reaction goes in a single step beyond arylammonium hydrogen sulphate to give the aminoaryl sulphonic acid.

9. Process according to Claims 5 to 8, characterized in that, in the concentric two-component nozzle, the acid component is sprayed through the central nozzle and the alkaline component is sprayed through the annular gap.

## Revendications

1. Procédé de fabrication de composés chimiques se présentant sous la forme d'une substance solide au départ de matières premières liquides, caractérisé en ce qu'on pulvérise les matières premières liquides à partir d'une tuyère concentrique à deux matières avec fente annulaire et une tuyère avancée au milieu, en sorte de mélanger les matières premières liquides dans les cônes de pulvérisation qui se compénètrent, en étant mises ainsi à réagir, en l'occurrence le cône de pulvérisation interne faisant un angle de 1° à 40° plus grand que le cône de pulvérisation externe, et en ce qu'on recueille les particules de matière solide obtenues dans un agent de suspension qui se trouve dans une cuve au-dessous des tuyères.

2. Procédé selon la revendication 1, caractérisé en ce que l'angle des cônes de pulvérisation est de 5 à 120°.

3. Procédé selon les revendications 1 et 2, caractérisé en ce que l'on engendre dans la tuyère une torsion du cône de pulvérisation.

4. Procédé selon les revendications 1 à 3, caractérisé en ce que la torsion des deux cônes de pulvérisation est engendrée dans des directions opposées.

5. Procédé de fabrication de sels alcalins d'acides sulfoniques aromatiques se présentant sous la forme d'une matière solide par réaction d'acides sulfoniques avec un hydroxyde alcalin selon la revendication 1, caractérisé en ce qu'on fait réagir des acides sulfoniques fondus avec un hydroxyde alcalin liquide au moyen d'une tuyère concentrique à deux matières, avec mélange et neutralisation qui ont lieu en dehors des tuyères.

6. Procédé suivant la revendication 5, pour la fabrication de suspensions contenant du sel sodique d'acide diphényl-4,4'-disulfonique convenant pour la fusion alcaline en 4,4'-dihydroxydiphényle, caractérisé en ce qu'on utilise comme sulfoacide le mélange technique de sulfonation comportant au moins 30 % en poids d'acide diphényl-4,4'-disulfonique et comme hydroxyde alcalin de l'hydroxyde de sodium aqueux à au moins 50 % en poids, en une quantité d'environ 8 à 24 moles de NaOH par mole de sel sodique d'acide diphényl-4,4'-disulfonique après la neutralisation de tous les groupes acides.

7. Procédé de fabrication d'hydrogénosulfates d'arylammonium à partir d'une arylamine et d'acide sulfurique selon la revendication 1, caractérisé en ce qu'on fait réagir l'arylamine fondue ou liquéfiée par addition de solvants et d'acide sulfurique contenant éventuellement de l'eau au moyen d'une tuyère concentrique à deux matières, avec mélange et neutralisation s'effectuant à l'extérieur des tuyères.

8. Procédé de fabrication d'acides aminoarylsulfoniques au départ d'arylamines et d'acides sulfuriques selon les revendications 1 et 7, caractérisé en ce qu'on fait réagir l'arylamine fondue ou liquéfiée par addition de solvants et d'acide sulfurique contenant éventuellement de l'eau au moyen d'une tuyère concentrique à deux matières, avec mélange et neutralisation s'effectuant à l'extérieur des tuyères, et en ce que pour le maintien d'une température de 120-180 °C on effectue la réaction en un stade avec passage par l'hydrogénosulfate d'arylammonium en vue d'obtenir l'acide aminoarylsulfonique.

9. Procédé selon les revendications 5 à 8, caractérisé en ce que dans la tuyère concentrique à deux matières on pulvérise le composant acide à travers la tuyère centrale et le composant alcalin à travers la fente annulaire.

FIG. 1